# EUROPEAN PATENT SPECIFICATION

(11) **EP 1 933 849 B1**
(45) Date of publication and mention of the grant of the patent: **26.10.2016**
(21) Application number: 06842320.1
(22) Date of filing: 29.09.2006
(51) Int. Cl.: A61K 31/7048, A61P 33/14, A61P 27/02

(54) **Use of ivermectin in the treatment of ophthalmic pathologies**
Verwendung von Ivermectin bei der Behandlung von Augenleiden
Utilisation d'ivermectine pour le traitement des pathologies ophtalmiques

(30) Priority: 30.09.2005 FR 0510025; 12.10.2005 US 725320 P; 05.07.2006 US 818316 P
(43) Date of publication of application: 25.06.2008
(60) Divisional application: 12152476.3
(73) Proprietor: Galderma S.A., 6330 Cham (CH)
(72) Inventor: KAOUKHOV, Alexandre, F-06160 Juan les Pins (FR); VILLARD, Christophe, F-06530 Le Tignet (FR); BOUISSOU, Philippe, F-06330 Roquefort Les Pins (FR)
(74) Representative: Casalonga
(86) International application number: PCT/IB2006/003864
(87) International publication number: WO 2007/054822

(56) References cited:
- US-A- 5 614 545
- US-A- 5 952 372
- US-A- 5 958 443
- US-A- 6 133 310
- US-A1- 2002 183 399
- US-A1- 2004 167 084
- WILHELMUS KIRK R: "ANTIPARASITIC DRUGS IN OPHTHALMOLOGY" INTERNATIONAL OPHTHALMOGY CLINICS, LITTLE, BROWN AND CO., BOSTON, US, vol. 36, no. 2, 1996, pages 117-152, XP008077587 ISSN: 0020-8167
- BIANCIARDI, P. ET AL: "Treatment of dog thelaziosis caused by Thelazia callipaeda (Spirurida, Thelaziidae) using a topical formulation of imidacloprid 10% and moxidectin 2.5%" VETERINARY PARASITOLOGY , 129(1-2), 89-93 CODEN: VPARDI; ISSN: 0304-4017, 2005, XP004828565
- MOLYNEUX D H ET AL: "Mass drug treatment for lymphatic filariasis and onchocerciasis" TRENDS IN PARASITOLOGY 2003 UNITED KINGDOM, vol. 19, no. 11, 2003, pages 516-522, XP008080195 ISSN: 1471-4922
- COTREAU M M ET AL: "The antiparasitic moxidectin: Safety, tolerability, and pharmacokinetics in humans." JOURNAL OF CLINICAL PHARMACOLOGY, vol. 43, no. 9, September 2003 (2003-09), page 1022, XP008080252 & THIRTY-SECOND ANNUAL MEETING OF THE AMERICAN COLLEGE OF CLINICAL PHARMACOLOGY; WESLEY CHAPEL, FLORIDA, USA; SEPTEMBER 21-23, 2003 ISSN: 0091-2700
- LOO W J ET AL: "INVERMECTIN CREAM IN ROSACEA: COMPARISON WITH METRONIDAZOLE GEL", BRITISH JOURNAL OF DERMATOLOGY, OXFORD : WILEY-BLACKWELL, UK, vol. 151, 1 July 2004 (2004-07-01), pages 61,ABSTR.NO.P-92, XP009056313, ISSN: 0007-0963
- "Stromectol 3 mg" In: Vidal: "VIDAL 2003 - Le Dictionnaire", 1 January 2003 (2003-01-01), Vidal publ., Paris pages 1781-1782,

## Description

The present invention relates to the use of ivermectin in the preparation of a pharmaceutical composition intended for the treatment of ocular rosacea.

Ocular rosacea is frequently diagnosed when it coexists with cutaneous signs or symptoms of rosacea (Browning D.J. and Proia A. D., Ocular Rosacea, Surv. Ophthalmol., 1986, 31, 145-58). Ocular rosacea is independent of the severity of rosacea of the skin (Donshik P. C, Hoss D.M. and Ehlers W.H., Inflammatory and papulosquamous disorders of the skin and eye, Dermatologic Clinics, 1992, 3, 533-47).

In the event of ocular attack, some studies show more women affected than men, whereas there is no significant difference according to sex for rosacea of the skin (Ramelet A.A., Rosacea: a reaction pattern associated with ocular lesions and headache, Arch. Dermatol., 1994, 130, 1448).

For a proportion of patients affected by ocular rosacea ranging up to 20%, the ocular signs and symptoms can occur before the cutaneous manifestations. The relevant symptoms for the diagnosis of ocular rosacea are as follows: feeling of burning or of smarting, feeling of a foreign body, feeling of dryness of the eye, increased sensitivity to light, blurred vision. The least serious clinical signs in patients affected by ocular rosacea are telangiectasia of the eyelid margin, meibomitis, chalazia, conjunctival hyperaemia and papillary conjunctivitis (Donshik P. C, Hoss D.M. and Ehlers W.H., Inflammatory and papulosquamous disorders of the skin and eye, Dermatologic Clinics, 1992, 3, 533-47). Dryness of the eye with quantitative deficiency of tears and bacterial superinfection frequently exist.

More serious are the problems posed by attacks on the cornea in the form of superficial punctuate keratitis and interstitial keratitis, which can develop into a decrease in visual acuity, ulceration or perforation (Jenkins M.S., Brown S. I., Lempert S .L. and Weinberg R.J., Ocular rosacea, Am. J. Ophthalmol., 1979, 88, 618-22).

The causes of the ocular pathologies mentioned above include in particular the presence of various organisms, such as *Demodex folliculorum,* the commonest human ectoparasite. However, not all ocular pathologies are related to the presence of this ectoparasite.

If ocular rosacea, which is included among ophthalmic pathologies, is not treated, serious complications may occur in the cornea and this can detrimentally affect the vision.

The therapeutic measures commonly used are the application of hot compresses and of topical antibiotics, such as a metronidazole gel, for example. A marked improvement is obtained but is often followed by a relapse in the form of conjunctivitis.

Oral antibiotics, such as tetracycline hydrochloride or doxycycline, can also be used. However, the level of relapse is of the order of 66% after treatment for 6 months (Zug K.A., Palay D.A. and Rock B., Dermatologic diagnosis and treatment of itchy red eyelids, Surv. Ophthalmol., 1996, 40, 293-306). A maintenance treatment is often necessary, sometimes for several years, indeed even for life. Cyclines have an anti-inflammatory action but do not have a curative action. The longer the treatment, the more there exists a risk of appearance of resistance (Quaterman M.J., Johnson D. W., Abele D. C, Lesher J.L., Hull D. S. and Davis L. S., Ocular rosacea, Arch. Dermatol., 1997, 133, 49-54). A loss in the anti∼inflammatory effectiveness is possible during long-term treatments, hence the interest in short cures (over 1 to 2 months). This treatment is used in first intention, during severe attacks on the eyes, due to the risk of aftereffects on the eyes.

An antibiotic of the class of the macrolides (erythromycin) can also be used but the response to the treatment is not systematic.

Furthermore, patients suffering from ocular rosacea must not take isotretinoin, as this substance aggravates ophthalmic symptoms (Michel J. L., Valanconny C, Gain P., Montelimard N., Tchaplyguine F. and Cambazard F., Manifestations oculaires des retinoides [Ocular manifestations of retinoids], Ann. Dermatol. Venereol., 1998, 125, 438-42).

None of these treatments makes possible complete and lasting remission of ocular rosacea. In view of the above, there thus exists a need to formulate a composition which shows an improved effectiveness in the treatment of ocular rosacea and which does not exhibit the side effects described in the prior art.

Ivermectin is a mixture of two compounds belonging to the class of the avermectins, 5-O-demethyl-22,23-dihydroavermectin A₁ₐ and 5-O-demethyl- 22, 23-dihydroavermectin A_{1b}. They are also known under the names of 22,23-dihydroavermectin B₁ₐ and 22,23-dihydroavermectin B_{1b}. Ivermectin comprises at least 80% of 22,23-dihydroavermectin B₁ₐ and less than 20% of 22,23-dihydroavermectin B_{1b}. This active agent forms part of the class of the avermectins, a group of macrocyclic lactones produced by the bacterium Streptomyces avermitilis (Reynolds J. E. F. (Ed), (1993) Martindale, The Extra Pharmacopoeia, 29th Edition, Pharmaceutical Press, London).

In the middle of the 1980s, ivermectin was presented as a broad-spectrum antiparasitic medicament for veterinary use (Campbell W. C. et al. (1983), Ivermectin: a potent new antiparasitic agent, Science, 221, 823-828). It is effective against the majority of common intestinal worms (except for the Teniae), the majority of the acarids and a few lice. It exhibits in particular a high affinity for the glutamate-dependent chloride channels present in the nerve and muscle cells of invertebrates. Its attachment to these channels promotes an increase in the membrane permeability to chloride ions, resulting in hyperpolarization of the nerve or muscle cell. This results in neuromuscular paralysis, which can bring about the death of certain parasites. Ivermectin also interacts with other ligand- dependent chloride channels, such as those involving the GABA (γ-aminobutyric acid) neuromediator.

Ivermectin is more particularly an anthelmintic. It is already described in man in the treatment of onchocerciasis due to Onchocerca volvulus, of gastrointestinal strongyloidiasis (product Stromectol ® and of human scabies (Meinking T. L. et al., N. Engl. J. Med., 1995, Jul., 6, 331(1), 26-30, The treatment of scabies with ivermectin) and in the treatment of microfilaraemia diagnosed or suspected in subjects affected by lymphatic filariasis due to Wuchereria bancrofti.

Moreover, Patent US 6 133 310 discloses the use of invermectin topically in the treatment of rosacea of the skin, in the form of a prototype of a lotion composed of a mixture of invermectin and of water, and also mentions the possibility of a prototype of a cream composed, for its part, of the mixture of invermectin and of an excipient, such as propylene glycol or sodium lauryl sulphate, but does not describe any- pharmaceutical composition as such. These mixtures are similar to experimental preparations to be applied to the skin, used in the context of first results of a proof of concept. Specifically, the facts disclosed in this patent do not teach a person skilled in the art anything regarding the feasibility and the effectiveness of pharmaceutical compositions acceptable industrially in the treatment of ocular rosacea.

The Applicant Company has discovered, surprisingly, that ivermectin is suitable for the treatment of ocular rosacea.

The term "treatment" is understood to mean, according to the invention, treatment in man.

A subject-matter of the present invention is thus the use of ivermectin in the preparation of a unique, non-irritating, pharmaceutical composition intended for the treatment of ocular rosacea.

The term "pharmaceutical composition" is understood to mean, according to the invention, a stable composition comprising a therapeutically active agent which has a good cosmetic quality and a satisfactory time limit (18 months minimum).

According to the invention, the term "unique pharmaceutical composition" is understood to mean that the composition comprising ivermectin is used alone to treat ocular rosacea ; combinations with other pharmaceutical compositions are thus excluded.

According to the present invention, ivermectin is the only active principle used in the composition for treating ocular rosacea; no active principle other than ivermectin, whether in particular administered topically or orally, is used according to the invention.

The pharmaceutical composition according to the invention, comprising ivermectin, is intended in particular for the treatment of ophthalmic symptoms, symptoms chosen from a feeling of burning or of smarting of the eye, a feeling of a foreign body in the eye, a feeling of dryness of the eye, an increased sensitivity to light, blurred vision, telangiectasia of the eyelid margin, meibomitis, chalazia, conjunctival hyperaemia and papillary conjunctivitis.

According to another aspect of the invention, the pharmaceutical composition according to the invention, comprising ivermectin is intended in particular for the treatment of conjunctivitis or blepharitis.

The pharmaceutical composition according to the invention is intended for the treatment of the eyes topically .

The topical application represents the commonest method of administration of ophthalmic medicaments. The topical route makes possible the application in the eye of eyewashes . This composition for topical application can be provided in anhydrous form, in aqueous form or in the form of an emulsion.

The pharmaceutical composition for topical use must be nonirritating and compatible with the tissues of the eye. The solutions are sterile preparations free from all particles.

The composition according to the invention preferably comprises from ivermectin, by weight with respect to the total weight of the composition.

The composition according to the invention is provided in the form of an eyewash. The term "eyewash" is understood to mean a liquid formulation specifically appropriate for administration to the conjunctiva of the eye and to the cornea. The eyewash is characterized by a volume of the instilled drops of approximately 25-50 microlitres.

As mentioned above, the compositions according to the invention have to meet specific conditions in order to be able to be applied in the eye. Such conditions include in particular sterility, absence of irritation and compatibility with the tissues of the eye. The latter criterion is more difficult to obtain than for a composition applied to the skin; in particular, compounds such as ethanol or glycols, formulated in compositions to be applied to the skin, cannot be present in compositions for ocular use. The topical compositions according to the invention make it possible to directly and specifically treat the symptoms of the pathology in the eye and eyelids by a local action; in particular, since only the eye is targeted, a better effectiveness can be expected. Furthermore, the topical compositions according to the invention preferably being the only ones administered to treat ocular rosacea, interactions between active principles can be reduced, indeed even avoided.

The pharmaceutical composition according to the invention can additionally comprise inert additives or combinations of these additives, such as
- wetting agents, emollients;
- agents for improving the flavour;
- preservatives;
- stabilizing agents;
- agents for regulating moisture;
- pH-regulating agents;
- buffers;
- agents for modifying osmotic pressure;
- emulsifying agents;
- agents for increasing the viscosity;
- and antioxidants.

Of course, a person skilled in the art will take care to choose the optional compound or compounds to be added to these compositions so that the advantageous properties intrinsically attached to the present invention are not, or not substantially, detrimentally affected by the envisaged addition. Various formulations of compositions comprising ivermectin will now be given, by way of illustration and without any limiting nature.

### EXAMPLE 1

| | |
|---|---|
| - Ivermectin | 0.03% |
| - Polysorbate 80 | 2.00% |
| - Benzalkonium chloride | 0.05% |
| - EDTA | 0.05% |
| - Water | q.s. for 100 |
| - Buffer system | pH 6.3 |

### EXAMPLE 2

| | |
|---|---|
| - Ivermectin | 0.10% |
| - Polysorbate 80 | 5.00% |
| - Phenylethyl alcohol | 0.50% |
| - Hydroxypropylcellulose | 1.20% |
| - Sorbitol | 2.00% |
| - Water | q.s. for 100 |
| - Monosodium phosphate/sodium sulphite heptahydrate (buffer system) | qs. pH 6.5 |

## Claims

1. Use of ivermectin as sole active principle in the preparation of a unique, non-irritating pharmaceutical composition for the treatment of ocular rosacea **characterized in that** the composition is administrated topically and is provided in the form of an eyewash; the composition comprising from 0.01 to 5% by weight of ivermectin with respect to the total weight of the composition.

2. Use according to Claim 1, **characterized in that** the pharmaceutical composition is for the treatment of symptoms chosen from a feeling of burning or of smarting of the eye, a feeling of a foreign body in the eye, a feeling of dryness of the eye, an increased sensitivity to light, blurred vision, telangiectasia of the eyelid margin, meibomitis, blepharitis, chalazia, conjunctival hyperaemia and papillary conjunctivitis.

3. Use according to one of Claims 1 to 2, **characterized in that** the pharmaceutical composition is for the treatment of blepharitis or conjunctivitis.

## Patentansprüche

1. Verwendung von Ivermectin als einzigem Wirkprinzip in der Herstellung einer einzigartigen, nicht-reizenden pharmazeutischen Zusammensetzung zur Behandlung von okulärer Rosazea, **dadurch gekennzeichnet, dass** die Zusammensetzung topisch verabreicht wird und in Form eines Augenbads bereitgestellt wird; wobei die Zusammensetzung in Bezug auf das Gesamtgewicht der Zusammensetzung 0,01 bis 5 Gew.% Ivermectin umfasst.

2. Verwendung nach Anspruch 1, **dadurch gekennzeichnet, dass** die pharmazeutische Zusammensetzung zur Behandlung von Symptomen ausgewählt aus einem Gefühl des Brennens oder Schmerzens des Auges, einem Fremdkörpergefühl im Auge, einem Trockenheitsgefühl des Auges, einer erhöhten Lichtempfindlichkeit, verschwommener Sicht, Telangiektasie des Augenlidrandes, Meibomitis, Blepharitis, Chalasie, konjunktivaler Hyperämie und Papillarkonjunktivitis dient.

3. Verwendung nach einem der Ansprüche 1 bis 2, **dadurch gekennzeichnet, dass** die pharmazeutische Zusammensetzung zur Behandlung von Blepharitis oder Konjunktivitis dient.

## Revendications

1. Utilisation d'ivermectine en tant que principe actif unique dans la préparation d'une composition pharmaceutique unique non irritante pour le traitement de la rosacée oculaire, **caractérisée en ce que** la composition est administrée localement et se présente sous la forme d'un lavage oculaire ; la composition comprenant de 0,01 à 5 % en poids d'ivermectine par rapport au poids total de la composition.

2. Utilisation selon la revendication 1, **caractérisée en ce que** la composition pharmaceutique est destinée au traitement de symptômes parmi une sensation de brûlure ou de picotement dans l'oeil, une sensation d'un corps étranger dans l'oeil, une sensation de sécheresse de l'oeil, une sensibilité accrue à la lumière, une vision floue, une télangiectasie du bord des paupières, la meibomite, la blépharite, les chalazions, l'hyperémie conjonctive et la conjonctivite papillaire.

3. Utilisation selon la revendication 1 ou 2, **caractérisée en ce que** la composition pharmaceutique est pour le traitement de la blépharite ou de la conjonctivite.
